**Europäisches Patentamt**

**European Patent Office**

**Office européen des brevets**

(11) Veröffentlichungsnummer: **0 294 851**
**A2**

## (12) EUROPÄISCHE PATENTANMELDUNG

(21) Anmeldenummer: **88109370.2**

(22) Anmeldetag: **13.06.88**

(51) Int. Cl.4: **C07K 1/02 , C12P 21/02 , //C07K7/40**

(30) Priorität: **12.06.87 DD 303735**

(43) Veröffentlichungstag der Anmeldung:
**14.12.88 Patentblatt 88/50**

(84) Benannte Vertragsstaaten:
**AT BE CH DE ES FR GB IT LI NL**

(71) Anmelder: **VEB Berlin-Chemie**
**Glienicker Weg 125-127**
**DDR-1199 Berlin(DD)**

(72) Erfinder: **Jakubke, Hans-Dieter, Prof.**
**Dr.sc.nat.**
**Johannes-R.-Becher-Strasse 4/1015**
**DD-7030 Leipzig(DD)**
Erfinder: **Könnecke, Andreas, Dr.rer.nat.**
**Kleiststrasse 39**
**DD-7022 Leipzig(DD)**
Erfinder: **Hänsicke, André, Dr.rer.nat.**
**Handjerystrasse 2**
**DD-1199 Berlin(DD)**
Erfinder: **Krause, Eberhard, Dr.rer.nat.**
**Robert-Uhrig-Strasse 18**
**DD-1136 Berlin(DD)**

(74) Vertreter: **Patentanwälte Beetz sen. - Beetz**
**jun. Timpe - Siegfried - Schmitt-Fumian-**
**Mayr**
**Steinsdorfstrasse 10**
**D-8000 München 22(DE)**

(54) **Verfahren zur Herstellung von Humaninsulin und seinen Derivaten.**

(57) Die Erfindung betrifft ein Verfahren zur semisynthetischen Herstellung von Humaninsulin und seinen Derivaten aus Schweineinsulin oder anderen sequenzentsprechenden Insulinpräkursoren, bei dem als Enzym Trypsin in immobilisierter Form eingesetzt wird, das nach Aktivitätsregenerierung mehrfach verwendet werden kann. Die Aktivität des trägergebundenen Trypsins muß mindestens 1500 U/g trockener Träger betragen, wobei die Reaktion in einem Medium aus einem wäßrigen Puffersystem und einem organischen Lösungsmittelgemisch bei pH 6,8 und 19 - 22 °C in Gegenwart von $Ca^{2+}$-Ionen durchgeführt wird. Nach beendeter Reaktion wird das trägerfixierte Trypsin durch Filtration abgetrennt, in wäßrig-saurem Milieu bei pH ≤ 3 regeneriert und kann nach folgender Äquilibrierung im Reaktionsmedium wiederverwendet werden.

In Abhängigkeit vom Medium sind über 50 Wiederverwendungen möglich.

## Verfahren zur Herstellung von Humaninsulin und seinen Derivaten

Die Erfindung betrifft ein Verfahren zur biotechnologischen Herstellung von Humaninsulin und seinen Derivaten, wobei als Ausgangsmaterial Schweineinsulin und/oder andere sequenzentsprechende Insulin-Präkursoren eingesetzt werden.

Humaninsulin wird international in der Medizin zunehmend zur Therapie der Diabetes Mellitus eingesetzt und soll aus immunologischen Gründen vorteilhafter als andere Insulinspezies sein.

Die Herstellung von Humaninsulin ist prinzipiell auf drei Wegen möglich:

1) Durch Chemosynthese; 2) durch Semisynthese and 3) durch gentechnische Herstellung.

Aus ökonomischen Gründen ist die chemische Totalsynthese gegenwärtig nicht vertretbar (vgl. H.-D. Jakubke, H. Jeschkeit: Aminosäuren, Peptide, Proteine; Akademie-Verlag, Berlin, 1982, S. 296-304). Die gentechnische Her stellung von Humaninsulin erfolgt entweder über die Stufe des Proinsulins oder durch separate Herstellung der A- und B-Kette und anschließende Kombination beider Ketten (vgl. F. Wengenmayer, Angew. Chem. 95 - (1983) 874).

Da sich Humaninsulin von Schweineinsulin nur durch die C-terminale Aminosäure der B-Kette (Threonin bzw. Alanin) unterscheidet, wurde nach Möglichkeiten gesucht, diesen Aminosäureaustausch semisynthetisch zu realisieren (vgl. H.-D. Jakubke et al., Angew. Chem. 97 (1985) 79). Zur direkten Umwandlung von Schweineinsulin in ein Humaninsulinderivat durch Inkubation von Schweineinsulin mit einem Threoninderivat unter Katalyse von Trpysin oder einer Protease ähnlicher Substratspezifität existiert Patentliteratur (EP 56 951, DE 31 04 949, DE 31 29 404, DD 160 282, WO 83 02 772).

Diese nur in den Reaktionsbedingungen differierenden bekannten Verfahren besitzen alle den Nachteil, daß der in ungewöhnlich hoher Konzentration einzusetzende Biokatalysator nur einmal genutzt werden kann. Es wurde ein Verfahren vorgeschlagen, bei dem unter schonenden Reaktionsbedingungen der Biokatalysator zurückgewonnen und wiederverwendet werden kann (DD 245 445).

Am effektivsten erscheint der Einsatz des Enzyms in immobilisierter Form.

Die existierenden Beschreibungen für Transpeptidierungen mit immobilisiertem Trypsin (DE 31 04 949, WO 83 02 772) setzen trägergebundenes Trypsin mit nur geringer Enzymaktivität ein (300 U/g bzw. 40-60 U/ml), so daß die für Transpeptidierungsreaktionen notwendigen hohen Enzymaktivitäten pro Volumeneinheit nicht erreicht werden und damit nur geringe Umsatzraten erzielt werden können. Es besteht deshalb die vorherrschende Meinung in der Fachwelt, daß immobilisiertes Trypsin für die direkte Umwandlung von Schweine- in Humaninsulin (Transpeptidierung) nicht geeignet ist. (K. Morihara et al., Biochem. J. 240 (1986) 803, zit. Lit.). Weiterhin fehlen Angaben zur Stabilität und Mehrfachverwendung für den Transpeptidierungsprozeß völlig.

Es ist das Ziel der Erfindung, Schweineinsulin und/oder andere entsprechende Insulinpräkursoren im Gegensatz zu den bekannten Verfahren unter Mehrfachverwendung des kovalent trägergebundenen Biokatalysators auf semisynthetischem Wege ökonomisch vorteilhafter in Humaninsulin und seine Derivate umzuwandeln.

Der Erfindung liegt die Aufgabe zugrunde, ein Verfahren anzugeben, mit dem die Umwandlung von Schweineinsulin oder anderer entsprechender Insulinpräkursoren in Humaninsulin oder seine Derivate unter Mehrfachverwendung von kovalent an einen unlöslichen, dabei vorzugsweise festen polymeren Träger (Polymermatrix) gebundenem Trypsin möglich ist.

Die Aufgabe wird gemäß Anspruch 1 gelöst. Die abhängigen Ansprüche betreffen vorteilhafte Ausführungsformen des Verfahrens.

Erfindungsgemäß erfolgt die enzymkatalysierte Umwandlung von Schweineinsulin oder anderer sequenzentsprechender Insulinpräkursoren in Humaninsulin oder seine Derivate in einem Gemisch aus einem organischen Lösungsmittel und einem spezifischen Puffer, in dem das trägerfixierte Enzym die geringstmögliche irreversible Inaktivierung erfährt, wodurch eine Mehrfachnutzung des Enzyms ermöglicht wird.

Erfindungsgemäß wird die Umwandlung vorteilhaft mit einem immobilisierten Trypsin vorgenommen, dessen Aktivität mindestens 1500 BAEE-U/g Träger bzw. dessen Volumenaktivität 200 U/ml Sediment beträgt.

Derart hoch mit Trypsin beladene Träger werden zum Beispiel durch Umsetzung von mit N-Chlorcarbonyloxy-5-norbornen-2,3-dicarboximid (Cl-CO-ONB) aktivierter Perlcellulose mit Trypsin erhalten (EP 134 041).

Eine solch hohe Aktivität ermöglicht die Realisierung einer dem löslichen Trypsin äquivalenten Aktivität im Ansatz, so daß die Reaktion in vergleichbarer Zeitdauer und mit vergleichbarer Umsatzrate abläuft. Vor der Wiederverwendung des immobilisierten Enzyms ist es erfindungsgemäß notwendig, dieses zur Regenerierung der vollen Aktivität zwischenzeitlich in ein wäßriges saures Milieu (pH $\leq$ 3) zu überführen.

Das Reaktionsmedium für die Umwandlung besteht aus einem Puffer und einem Lösungsmittelgemisch im Volumenverhältnis 45:55 bis 15:85. Als Puffersubstanz werden Tris oder Borsäure unter Zusatz von $CaCl_2$ eingesetzt. Das Lösungsmittelgemisch setzt sich aus 50 bis 70 % eines zweiwertigen Alkohols, vorzugsweise 1,3- oder 1,4-Butandiol, und 30 bis 50 % DMSO zusammen.

Das immobilisierte Trypsin wird vorzugsweise vor der Verwendung mit dem Reaktionsmedium äquilibriert.

Der Träger ist vorzugsweise ein Polysaccarid.

Die Umwandlung wird bei 19 bis 22 °C vorgenommen, wobei der apparente pH-Wert der Reaktionsmischung $pH_{app}$ 6,8 ± 0,2 beträgt und das Ausbeuteoptimum an Humaninsulinderivat innerhalb von 18 h erreicht wird. Erfindungsgemäß wird das trägerfixierte Enzym durch Filtration abgetrennt, wodurch gleichzeitig die Umwandlungsreaktion gestoppt wird.

Im Gegensatz zu bekannten Verfahren zur Umwandlung von Schweineinsulin in Humaninsulin wird durch die erfindungsgemäße Lösung eine ökonomisch effiziente Mehrfachnutzung des trägergebundenen Biokatalysators möglich. Gleichzeitig wird durch die einfache Abtrennbarkeit des gebundenen Enzyms eine Kontamination des Endproduktes mit proteolytischer Restaktivität und/oder immunogenem Enzymprotein ausgeschlossen.

Die Erfindung wird nachstehend anhand von Ausführungsbeispielen erläutert.

**Beispiel 1**

1,0 g kristallines Schweineinsulin und 1,3 g Threoninethylester werden in 3,2 ml Dimethylsulfoxid, 4,8 ml Butan-1,3-diol, 1 ml 0,25 M Tris-puffer pH 6,6 (10 mM $CaCl_2$) und 1 ml 6N HCl gelöst. Dazu werden 5 g immobilisiertes Trypsin an Perlcellulose mit einer Aktivität von 345 I.U./g feuchter Träger, (BAEE) äquilibriert mit einem Gemisch aus 16 ml DMSO, 24 ml Butan-1,3-diol und 10 ml 0,25 M Tris-Puffer pH 6,6 (10 mM $CaCl_2$) gegeben und bei 20 °C unter leichtem Schütteln inkubiert. Nach 5 h beträgt die Ausbeute an Humaninsulinethylester nach HPLC-Analyse 92 %. Anschließend wird vom immobilisierten Trypsin abgesaugt, das dreimal mit 10 ml DMSO-Wasser (8:2/V:V) ge waschen wird; die gesammelten Lösungen werden mit Aceton gefällt. Aus dem Fällungsprodukt wird das Humaninsulinderivat in bekannter Weise isoliert und nach Spaltung des Esters und nochmaliger Reinigung Humaninsulin erhalten.

Der Träger mit dem immobilisierten Trypsin wird mit Wasser und 0,01 N HCl gewaschen und anschließend unter 0,001 N HCl (10 mM $CaCl_2$) bei pH 5 über Nacht kühl gelagert und kann nach Äquilibrierung mit dem Transpeptidierungslösungsmittelgemisch im nächsten Transpeptidierungszyklus wieder eingesetzt werden.

**Beispiel 2**

Es wird wie in Beispiel 1 verfahren, wobei 1,6 g Threonin-t-butylester eingesetzt werden. Nach 5 h beträgt die Ausbeute an Humaninsulin-t-butylester nach HPLC-Analyse 90 %.

**Beispiel 3**

Es wird wie in Beispiel 1 verfahren, wobei 5 g an Perlcellulose immobilisiertes Trypsin verwendet wird, das in 15 Transpeptidierungszyklen eingesetzt worden war und eine Aktivität von 330 I.U./g feuchter Träger (BAEE) besitzt. Nach 5 h beträgt die Ausbeute an Humaninsulinethylester nach HPLC-Analyse 91 %.

**Ansprüche**

1. Verfahren zur Herstellung von Humaninsulin und seinen Derivaten, bei dem Schweineinsulin oder andere sequenzentsprechende Insulinpräkursoren in Gegenwart eines Biokatalysators mit einem Threoninderivat umgesetzt werden, **dadurch gekennzeichnet, daß** die Umsetzung in einem Reaktionsmedium aus 55 bis 85 Volumenteilen eines organischen Lösungsmittelgemisches, das aus 30 bis 50 % DMSO und 50 bis 70 % eines zweiwertigen Alkohols besteht, und einem wäßrigen Puffer bei einem apparenten pH-Wert $pH_{app}$ von 6,8 ± 0,2 und einer Temperatur von 19 bis 22 °C durchgeführt und als Biokatalysator immobilisiertes Trypsin eingesetzt wird.

2. Verfahren nach Anspruch 1, dadurch gekennzeichnet, daß ein immobilisiertes Trypsin eingesetzt wird, das kovalent an eine Polymermatrix gebunden ist.

3. Verfahren nach Anspruch 1 oder 2, dadurch gekennzeichnet, daß ein immobilisiertes Trypsin eingesetzt wird, das an ein unlösliches Polymer gebunden ist.

4. Verfahren nach einem der Ansprüche 1 bis 3, dadurch gekennzeichnet, daß ein immobilisiertes Trypsin eingesetzt wird, das an ein Polysaccharid als Träger gebunden ist.

5. Verfahren nach einem der Ansprüche 1 bis 4, dadurch gekennzeichnet, daß ein immobilisiertes Trypsin eingesetzt wird, dessen Aktivität mindestens 1500 BAEE-U/g trockener Träger beträgt.

6. Verfahren nach einem der Ansprüche 1 bis 5, dadurch gekennzeichnet, daß das immobilisierte Trypsin mehrfach eingesetzt wird.

7. Verfahren nach einem der Ansprüche 1 bis 6, dadurch gekennzeichnet, daß vor der Wiederverwendung die enzymatische Aktivität des immobilisierten Trypsins durch mindestens 5 h Behandlung in einem wässerigen Milieu bei pH ≤ 3 regeneriert wird.

8. Verfahren nach einem der Ansprüche 1 bis 7, dadurch gekennzeichnet, daß das immobilisierte Trypsin vor der Verwendung mit dem Reaktionsmedium äquilibriert wird.

9. Verfahren nach einem der Ansprüche 1 bis 8, dadurch gekennzeichnet, daß als zweiwertiger Alkohol 1,3- und/oder 1,4-Butandiol eingesetzt werden.